# FASCICULE DE BREVET EUROPEEN

(11) **EP 3 297 560 B1**
(45) Date de publication et mention de la délivrance du brevet: **24.04.2019**
(21) Numéro de dépôt: 16727743.3
(22) Date de dépôt: 18.05.2016
(51) Int. Cl.: A61B 18/14

(54) **DISPOSITIF POUR RÉALISER LA RÉSECTION D'UN ORGANE DANS UNE CAVITÉ D'UN CORPS**
VORRICHTUNG ZUR RESEKTION EINES ORGANS IN EINEM HOHLRAUM EINES KÖRPERS
DISPOSITIF POUR REALISER LA RESECTION D'UN ORGANE DANS UNE CAVITE D'UN CORPS

(30) Priorité: 20.05.2015 FR 1501040
(43) Date de publication de la demande: 28.03.2018
(73) Titulaire: AB Medica, 18100 Mery-sur-Cher (FR)
(72) Inventeur: BLANC Alexandre, 13013 Marseille (FR)
(74) Mandataire: Flavenot, Bernard
(86) Numéro de dépôt international: PCT/FR2016/000087
(87) Numéro de publication internationale: WO 2016/185102

(56) Documents cités:
- CA-A- 989 215
- DE-A1- 2 746 983
- FR-A1- 2 294 679
- US-B1- 6 358 200
- US-B1- 6 730 081

## Description

La présente invention concerne les dispositifs pour réaliser la résection de tout ou partie d'un organe situé dans une cavité d'un corps vivant, notamment d'un être humain, quelle que soit la nature de cet organe, qu'il soit sain ou non, et plus particulièrement les dispositifs connus dans le domaine sous la terminologie "résectoscopes".

De tels dispositifs sont par exemple décrits et illustrés dans les demandes de brevet WO2014114600, DE102009025047, CN201529144 et notamment le US 6 358 200.

Un tel dispositif connu comporte essentiellement : un guide défini entre une extrémité proximale et une extrémité distale, ce guide comprenant un conduit traversant débouchant à ses deux extrémités ; deux fils électriquement conducteurs, isolés extérieurement et montés coulissants dans le conduit, ces deux fils ayant chacun des première et seconde extrémités aptes à émerger, lors de leur translation dans le conduit, respectivement des deux extrémités proximale et distale du conduit, les premières extrémités des deux fils comportant des moyens pour une connexion à deux pôles d'une source d'énergie électrique ; une boucle ouverte définie entre deux bornes libres et réalisée en un matériau résistant électriquement conducteur pour produire de la chaleur par effet Joule et ainsi permettre, par brûlure, l'ablation de tout ou partie de l'organe déterminé; et des moyens pour connecter électriquement les deux bornes libres de la boucle ouverte respectivement aux deux secondes extrémités des deux fils conducteurs.

A l'intérieur de ce guide, sont en outre prévus des tubes permettant le passage, par exemple, d'instruments chirurgicaux ou analogues, l'introduction ou l'insufflation, dans la cavité, de différents fluides notamment gazeux, l'aspiration de tout élément présent dans la cavité, etc.

Il est en outre impératif que le guide qui doit être introduit dans la cavité (ou lumen) dans laquelle doit s'effectuer la résection soit d'une dimension transversale la plus faible possible et comporte une enveloppe extérieure qui puisse être facilement obtenue industriellement et commercialement, avec en outre l'obligation de contenir un nombre non négligeable de tubes et éléments indispensables pour réaliser la résection et éventuellement d'autres opérations chirurgicales que la résection.

Aussi, la présente invention a-t-elle pour but de réaliser un dispositif pour effectuer, comme défini ci-avant, la résection de tout ou partie d'un organe dans une cavité d'un corps vivant, notamment d'un être humain, qui réponde aux exigences mentionnées ci-dessus et qui puisse être facilement réalisé industriellement.

Plus précisément, la présente invention a pour objet un dispositif pour réaliser la résection d'un organe dans une cavité d'un corps vivant, notamment d'un être humain, connu sous le terme de "résectoscope", comportant au moins : un guide défini entre une extrémité proximale et une extrémité distale, ledit guide définissant un conduit débouchant à ses deux extrémités proximale et distale, ledit guide étant constitué par au moins un premier tube et un deuxième tube, tous deux cylindriques, le deuxième tube étant monté dans le premier tube, au moins un conducteur électrique entouré d'une gaine en un matériau électriquement isolant, ce dit conducteur électrique isolé étant monté dans ledit conduit, et des moyens d'entrée/sortie montés en coopération avec l'extrémité proximale du dit guide, caractérisé par le fait que ledit premier tube est cylindrique de révolution de rayon intérieur de valeur R1, ledit deuxième tube comporte : une première partie d'angle au centre d'une valeur Ac et cylindrique de révolution de rayon extérieur de valeur R2, R2 étant inférieure à R1, et une seconde partie cylindrique non de révolution et d'angle au centre de valeur "360° - Ac", les deux extrémités de cette seconde partie du deuxième tube étant en continuité respectivement des deux extrémités de la première partie de ce deuxième tube, ledit conducteur isolé étant monté au contact sur ladite seconde partie du dit deuxième tube, entre la paroi extérieure de ce deuxième tube et la paroi intérieure du premier tube, et qu'il comporte en outre des moyens pour maintenir ledit conducteur isolé au contact sur la seconde partie du deuxième tube.

D'autres caractéristiques et avantages de la présente invention apparaîtront au cours de la description suivante donnée en regard des dessins annexés à titre illustratif mais nullement limitatif, dans lesquels :
La figure 1 est une vue générale de côté d'un dispositif selon l'invention pour réaliser la résection de tout ou partie d'un organe dans une cavité d'un corps vivant, notamment d'un être humain, comportant un guide et des moyens d'entrée/sortie montés en association avec ce guide, et
La figure 2 est une vue en coupe d'un mode de réalisation du guide entrant dans la constitution du dispositif selon l'invention.

Il est précisé que, dans la présente description, si l'adverbe "sensiblement" est associé à un qualificatif d'un moyen donné, ce qualificatif doit être compris au sens strict ou approché.

La présente invention se rapporte à un dispositif pour réaliser la résection, par brûlure, d'un organe dans une cavité d'un corps vivant, notamment d'un être humain, qui trouve une application particulièrement avantageuse dans le domaine de l'hystéroscopie pour la chirurgie intra-utérine. Un tel dispositif connu des praticiens sous le terme "résectoscope" permet la réalisation d'interventions intra-utérines pour réaliser l'ablation de tout ou partie d'un organe, par exemple un polype, une tumeur, une synéchie, une malformation utérine ou analogue.

Ce dispositif en référence aux deux figures annexées comporte au moins un guide 10 défini entre une extrémité proximale 11 et une extrémité distale 12, ce guide définissant un conduit 13 débouchant à ses deux extrémités proximale et distale, le guide étant constitué par au moins un premier tube 21 et un deuxième tube 22, tous deux cylindriques, le deuxième tube 22 étant monté dans le premier tube 21, au moins un conducteur électrique entouré d'une gaine 33, 34 en un matériau électriquement isolant, ce conducteur électrique ainsi isolé 31, 32 étant monté dans le conduit 13, et des moyens d'entrée/sortie 14 montés en coopération avec l'extrémité proximale 11 du guide 10.

Selon une caractéristique essentielle de l'invention, le premier tube 21 est cylindrique de révolution de rayon intérieur de valeur R1, tandis que le deuxième tube 22 comporte une première partie 221 cylindrique de révolution de rayon extérieur de valeur R2 inférieure à R1 et d'angle au centre d'une valeur Ac, par exemple comme illustré sensiblement de 180°, et une seconde partie 222 cylindrique non de révolution et d'angle au centre de la valeur "360° - Ac", les deux extrémités 222-1, 222-2 de cette seconde partie 222 du deuxième tube 22 étant en continuité respectivement des deux extrémités de la première partie 221 de ce deuxième tube 22.

Le tube 21 qui est défini ci-dessus sera toujours désigné, pour faciliter la présente description, sous la terminologie de "tube cylindrique de révolution". Mais il est bien compris, au sens de la présente description, que cette terminologie recouvre aussi bien un tube cylindrique de révolution au vrai sens mathématique qu'un tube qui peut être assimilé, au moins fonctionnellement, à un tube cylindrique de révolution.

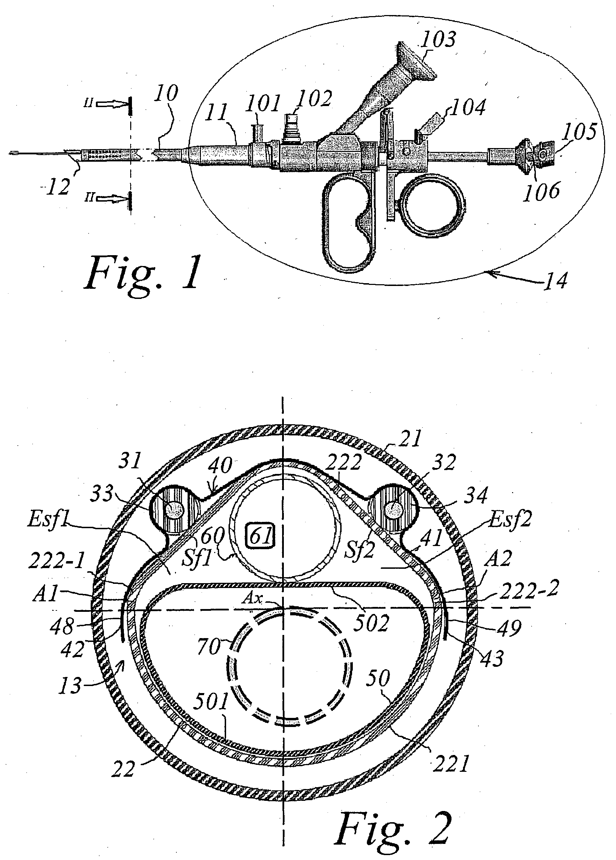

En outre, comme illustré sur la figure 2, le au moins un conducteur isolé 31, 32 est monté au contact sur la seconde partie 222 du deuxième tube 22, entre la paroi extérieure de ce deuxième tube et la paroi intérieure du premier tube 21, et le dispositif comporte des moyens 40 pour maintenir ce au moins un conducteur isolé 31, 32 au contact sur cette seconde partie 222 du deuxième tube 22.

Selon une caractéristique préférentielle de l'invention, les moyens 40 pour maintenir le conducteur isolé 31, 32 au contact, pas obligatoirement fixe mais pouvant être glissant selon son axe longitudinal, sur la seconde partie 222 du deuxième tube 22 sont constitués par un clip 41 en un matériau métallique relativement élastique comme une plaque préformée réalisée dans un matériau comme de l'acier inoxydable. Ce clip comporte alors deux bords longitudinaux latéraux 48, 49, chacun en forme de crochet élastique rentrant 42, 43 (c'est-à-dire présentant une forme sensiblement concave). Ces deux crochets élastiques rentrants sont clipsés en surmontant élastiquement les deux arêtes de liaison A1, A2 formées respectivement entre la première partie 221 du deuxième tube 22 et sa seconde partie 222, et en s'enroulant au moins partiellement autour de ces deux arêtes, le au moins un conducteur isolé 31, 32 étant relativement pincé entre ce clip 41 et la seconde partie 222 du deuxième tube 22, figure 2.

Selon une réalisation préférentielle, la seconde partie 222 du deuxième tube 22 présente, en regard de la paroi intérieure du premier tube 21, au moins une surface sensiblement plane Sf1, Sf2. Cette surface plane peut être facilement obtenue, soit à partir d'un tube classique cylindrique de révolution dont une partie de paroi est aplatie, soit directement par profilage.

De façon avantageuse, pour répondre à presque toutes les possibilités d'utilisation du dispositif, la seconde partie 222 du deuxième tube 22 présente, en regard de la paroi intérieure du premier tube, deux surfaces Sf1, Sf2 sensiblement planes faisant entre elles un angle compris entre 0° et 180°, limites non comprises, ces deux surface planes Sf1, Sf2 étant inscrites dans l'enveloppe de révolution définie par la surface extérieure de la première partie 221 du deuxième tube 22. De façon encore plus avantageuse, les deux surfaces sensiblement planes Sf1, Sf2 font entre elles un angle sensiblement égal à 90°, figure 2.

Dans ce cas, le dispositif peut alors très avantageusement comporter deux conducteurs isolés 31, 32 maintenus au contact respectivement sur chacune des deux surfaces sensiblement planes Sf1, Sf2, à l'aide du même clip 41 que celui décrit ci-avant.

Dans la configuration décrite ci-dessus, le dispositif peut alors aussi comporter avantageusement un troisième tube cylindrique 50, ce troisième tube comportant une première partie 501 cylindrique de révolution d'angle au centre d'une valeur sensiblement égale à 180° et de rayon extérieur de valeur R3 sensiblement égale à la valeur du rayon de la surface intérieure cylindrique de révolution de la première partie 221 du deuxième tube 22, et une seconde partie 502 sensiblement plane, les deux extrémités de cette seconde partie du troisième tube étant en continuité respectivement des deux extrémités de la première partie 501 de ce troisième tube, le troisième tube 50 étant disposé à l'intérieur du deuxième tube 22 de façon que sa première partie 501 soit sensiblement en congruence et en correspondance avec la surface intérieure de la première partie 221 du deuxième tube 22, comme illustré sur la figure 2.

Selon une réalisation qui est possible avec sa configuration décrite ci-dessus, le dispositif peut alors avantageusement comporter en outre un quatrième tube 60 cylindrique optionnellement de révolution, situé dans l'espace 61 défini entre la seconde partie 502 sensiblement plane du troisième tube 50 et les deux surfaces planes Sf1, Sf2 du deuxième tube 22.

Pour certaines applications, il est également avantageux de prévoir un cinquième tube 70, représenté en traits interrompus sur la figure 2, monté dans le troisième tube 50.

Selon une réalisation préférentielle sur le plan industriel, le premier tube 21, la première partie 221 du deuxième tube 22 et la première partie 501 du troisième tube 50, tous trois de forme cylindrique de révolution, sont sensiblement coaxiaux Ax, figure 2.

Les moyens d'entrée/sortie 14 mentionnés auparavant et illustrés sur la figure 1 sont connus de l'art antérieur et sont montés en relation, soit fluidiquement soit mécaniquement, avec les différents tubes et éléments qui ont été définis ci-avant.

C'est ainsi que, avec le dispositif selon l'invention dont un mode de réalisation est illustré sur les figures 1 et 2 :
- l'espace compris entre le premier tube 21 et le deuxième tube 22 est utilisé pour réaliser une aspiration lors d'une intervention chirurgicale, cette aspiration ayant une sortie au niveau de la bouche d'extraction 101 ;
- pour que la scène de l'opération à l'intérieur du lumen puisse être visible, il est nécessaire d'avoir, au niveau de la sortie distale 12 du guide 10, un éclairage lumineux. De façon avantageuse et connue, cet éclairage est fourni par un faisceau de fibres optiques monté en coopération avec une source lumineuse (non représentée sur les figures). Ce faisceau est situé dans l'espace en deux parties Esf1, Esf2 délimité par le deuxième tube 22, le troisième tube 50 et le quatrième tube 60. Ce faisceau de fibres optiques est généralement constitué en deux branches situées de part et d'autre du quatrième tube 60, essentiellement dans les deux parties Esf1 et Esf2, et émergeant par la bouche de sortie 102 ;
- comme dans toute intervention, afin de pouvoir observer le cours de l'opération, il est nécessaire d'introduire une caméra endoscopique. Cette introduction se fait par l'oeilleton d'entrée 103 qui est relié au quatrième tube 60, la caméra longeant à l'intérieur de ce quatrième tube 60 pour émerger à l'extrémité distale 12 du guide 10. Mais il est à noter que, à la place d'une caméra endoscopique, il est possible de disposer, dans ce tube 60, des éléments optiques, lentilles, guides d'ondes, etc. pour transmettre des images de la scène d'opération, et d'installer la caméra uniquement en coopération avec l'oeilleton d'entrée 103 ;

- comme mentionné ci-avant au préambule, un tel dispositif permet de réaliser des résections par découpe de corps, comme des polypes, situés dans un lumen, au moyen d'une résistance chauffée par effet Joule. Dans ce cas, les conducteurs électriques 31, 32, et éventuellement le premier tube 21, sont aptes à être reliés à une source d'énergie électrique. Les sorties des deux conducteurs 31, 32 sont situées sur la borne/prise électrique 104 ;
- lorsqu'il est nécessaire d'utiliser des instruments spécifiques autres que ceux pour une résection, ces instruments sont introduits sur le champ de l'opération par l'entrée 105 du dispositif qui comporte alors le cinquième tube 70 monté en coopération avec cette entrée qui se trouve dans l'axe Ax du guide 10, ce qui facilite l'introduction de ces instruments qui sont en général relativement rigides et qui de ce fait ne peuvent être introduits et glissés qu'en ligne droite ;
- l'intérieur du troisième tube 50 est utilisé pour envoyer, au niveau de l'intervention chirurgicale, un fluide d'irrigation qui est introduit par l'entrée 106 et qui est aspiré avec les déchets générés par l'opération, via l'espace relié à la sortie 101 définie ci-avant (figure 1).

Enfin, il est mentionné que les tubes 21, 22, 50 et 60 sont avantageusement réalisés en acier inoxydables, que les conducteurs électriques isolés 31, 32 sont généralement en un matériau électriquement bon conducteur, comme du cuivre, et que le matériau isolant dans lequel sont réalisées les gaines 33, 34 est par exemple du PTFE (polytétrafluoroéthylène).

Le mode d'utilisation du dispositif décrit ci-dessus est bien connu en lui-même. Il ne sera donc pas plus amplement développé ici dans l'unique souci de simplifier la présente description, d'autant plus que cette utilisation n'entre pas dans le champ de protection de l'invention.

Il sera cependant souligné que, par sa structure, le dispositif selon l'invention tel que décrit ci-dessus est très compact et peu encombrant transversalement tout en offrant aux Praticiens un maximum de possibilités d'utilisation, même pour des interventions chirurgicales dans des cavités d'accès relativement étroits et délicats.

## Revendications

1. Dispositif pour réaliser la résection d'un organe dans une cavité d'un corps vivant, notamment d'un être humain, connu sous le terme de "résectoscope", comportant au moins :
• un guide (10) défini entre une extrémité proximale (11) et une extrémité distale (12), ledit guide définissant un conduit (13) débouchant à ses deux extrémités proximale et distale, ledit guide étant constitué par au moins un premier tube (21) et un deuxième tube (22), tous deux cylindriques, le deuxième tube (22) étant monté dans le premier tube (21),
• au moins un conducteur électrique entouré d'une gaine (33, 34) en un matériau électriquement isolant, ce dit conducteur électrique ainsi isolé (31, 32) étant monté dans ledit conduit (13), et
• des moyens d'entrée/sortie (14) montés en coopération avec l'extrémité proximale (11) du dit guide (10),
**caractérisé par le fait que** :
• ledit premier tube (21) est cylindrique de révolution de rayon intérieur de valeur R1,
• ledit deuxième tube (22) comporte :
* une première partie (221) d'angle au centre d'une valeur Ac, et cylindrique de révolution de rayon extérieur de valeur R2, R2 étant inférieure à R1, et
* une seconde partie (222) cylindrique non de révolution et d'angle au centre de la valeur "360° -. Ac", les deux extrémités (222-1, 222-2) de cette seconde partie (222) du deuxième tube (22) étant en continuité respectivement des deux extrémités de la première partie (221) de ce deuxième tube,
• ledit conducteur isolé (31, 32) étant monté au contact sur ladite seconde partie (222) du dit deuxième tube (22), entre la paroi extérieure de ce deuxième tube et la paroi intérieure du premier tube (21), et
• qu'il comporte en outre des moyens (40) pour maintenir ce dit conducteur isolé (31, 32) au contact sur cette seconde pa,rtie (222) du deuxième tube (22).

2. Dispositif selon la revendication 1, **caractérisé par le fait que** les moyens (40) pour maintenir ledit conducteur isolé (31, 32) au contact sur la seconde partie (222) du deuxième tube (22) sont constitués par un clip (41) comportant deux bords longitudinaux latéraux, chacun en forme de crochet élastique rentrant (42, 43), ces deux crochets élastiques rentrants étant clipsés en surmontant les deux arêtes de liaison (A1, A2) formées respectivement entre la première partie (221) du deuxième tube (22) et sa seconde partie (222), et en s'enroulant au moins partiellement autour de ces deux arêtes, ledit conducteur isolé (31, 32) étant positionné entre ce clip (41) et ladite seconde partie (222) du deuxième tube (22).

3. Dispositif selon l'une des revendications 1 et 2, **caractérisé par le fait que** ladite seconde partie (222) du deuxième tube (22) présente, en regard de la paroi intérieure du premier tube (21), au moins une surface sensiblement plane (Sf1, Sf2).

4. Dispositif selon la revendication 3, **caractérisé par le fait que** ladite seconde partie (222) du deuxième tube (22) présente, en regard de la paroi intérieure du premier tube, deux surfaces (Sf1, Sf2) sensiblement planes faisant entre elles un angle compris entre 0° et 180°, limites non comprises, ces deux surface planes (Sf1, Sf2) étant inscrites dans l'enveloppe de révolution définie par la surface extérieure de la première partie (221) du deuxième tube (22).

5. Dispositif selon la revendication 4, **caractérisé par le fait que** les deux surfaces sensiblement planes (Sf1, Sf2) font entre elles un angle sensiblement égal à 90°.

6. Dispositif selon l'une des revendications 4 et 5, **caractérisé par le fait qu'**il comporte deux conducteurs isolés (31, 32) maintenus au contact par ledit clip (41) respectivement sur chacune des deux surfaces sensiblement planes (Sf1, Sf2).

7. Dispositif selon l'une des revendications précédentes, **caractérisé par le fait qu'**il comporte un troisième tube cylindrique (50), ce dit troisième tube comportant :
• une première partie (501) cylindrique de révolution d'angle au centre d'une valeur sensiblement égale à 180° et de rayon extérieur de valeur R3 sensiblement égale à la valeur du rayon de la surface intérieure cylindrique de révolution de la première partie (221) du deuxième tube (22),
• une seconde partie (502) sensiblement plane, les deux extrémités de cette seconde partie du troisième tube étant en continuité respectivement des deux extrémités de ladite première partie (501) du dit troisième tube,
• ledit troisième tube (50) étant disposé à l'intérieur du deuxième tube (22) de façon que sa première partie (501) soit sensiblement en congruence et en correspondance avec la surface intérieure de la première partie (221) du deuxième tube (22).

8. Dispositif selon la revendication 7 quand elle dépend de la revendication 5, **caractérisé par le fait qu'**il comporte un quatrième tube (60) cylindrique optionnellement de révolution, situé dans l'espace (61) défini entre la seconde partie (502) sensiblement plane du troisième tube (50) et les deux surfaces planes (Sf1, Sf2) du deuxième tube (22).

9. Dispositif selon l'une des revendications 7 et 8, **caractérisé par le fait qu'**il comporte un cinquième tube (70) monté dans ledit troisième tube (50).

10. Dispositif selon les revendications 1 et 7, **caractérisé par le fait que** ledit premier tube (21), la première partie (221) du deuxième tube (22) et la première partie (501) du troisième tube (50), tous trois de forme cylindrique de révolution, sont sensiblement coaxiaux (Ax).

## Patentansprüche

1. Vorrichtung für die Durchführung der Resektion eines Organs in einem Hohlraum eines lebenden Körpers, insbesondere eines Menschen, bekannt unter dem Ausdruck "Resektoskop" und und mindestens umfassend:
• eine Führung (10), die zwischen einem proximalen Ende (11) und einem distalen Ende (12) definiert ist, wobei die Führung eine Leitung (13) definiert, die an ihrem proximalen und an ihrem distalen Ende offen ist, wobei die Führung mindestens durch ein erstes Rohr (21) und ein zweites Rohr (22) gebildet ist, die beide zylindrisch sind, wobei das zweite Rohr (22) in dem ersten Rohr (21) montiert ist,
• mindestens einen elektrischen Leiter, der von einer Hülle (33, 34) aus einem elektrisch isolierenden Material umgeben ist, wobei der auf diese Weise isolierte elektrische Leiter (31, 32) in der Leitung (13) montiert ist, und
• Eingangs-/Ausgangsmittel (14), die in Zusammenwirkung mit dem proximalen Ende (11) der Führung (10) montiert sind,
**dadurch gekennzeichnet, dass**:
• das erste Rohr (21) rotationssymmetrisch zylindrisch mit einem Innenradius mit Wert R1 ist,
• das zweite Rohr (22) umfasst:
* einen ersten Teil (221) mit einem Zentrumswinkel mit Wert Ac, der rotationssymmetrisch zylindrisch mit einem Außenradius mit Wert R2 ist, wobei R2 kleiner als R1 ist, und
* einen zweiten, nicht rotationssymmetrischen zylindrischen Teil (222) mit einem Zentrumswinkel mit Wert "360° - Ac", wobei die beiden Enden (222-1, 222-2) dieses zweiten Teils (222) des zweiten Rohrs (22) in die beiden entsprechenden Enden des ersten Teils (221) dieses zweiten Rohrs übergehen,
• wobei der isolierte Leiter (31, 32) in Kontakt mit dem zweiten Teil (222) des zweiten Rohrs (22) zwischen der Außenwand dieses zweiten Rohrs und der Innenwand des ersten Rohrs (21) montiert ist und
• dass sie weiterhin Mittel (40) aufweist, um den isolierten Leiter (31, 32) in Kontakt mit diesem zweiten Teil (222) des zweiten Rohrs (22) zu halten.

2. Vorrichtung nach Anspruch 1, **dadurch gekennzeichnet, dass** die Mittel (40) zum Halten des isolierten Leiters (31, 32) in Kontakt mit dem zweiten Teil (222) des zweiten Rohrs (22) durch einen Clip (41) mit zwei seitlichen längsverlaufenden Rändern gebildet sind, von denen jeder einen zurückspringenden elastischen Haken (42, 43) bildet, wobei diese beiden zurückspringenden elastischen Haken über die beiden Verbindungskanten (A1, A2), die zwischen dem ersten Teil (221) des zweiten Rohrs (22) und seinem zweiten Teil (222) gebildet sind, überstehend einrasten und sich mindestens teilweise um diese zwei Kanten wickeln, wobei der isolierte Leiter (31, 32) zwischen diesem Clip (41) und dem zweiten Teil (222) des zweiten Rohrs (22) positioniert ist.

3. Vorrichtung nach einem der Ansprüche 1 und 2, **dadurch gekennzeichnet, dass** der zweite Teil (222) des zweiten Rohrs (22) gegenüber der Innenwand des ersten Rohrs (21) mindestens eine im Wesentlichen ebene Fläche (Sf1, Sf2) aufweist.

4. Vorrichtung nach Anspruch 3, **dadurch gekennzeichnet, dass** der zweite Teil (222) des zweiten Rohrs (22) gegenüber der Innenwand des ersten Rohrs zwei im Wesentlichen ebene Flächen (Sf1, Sf2) aufweist, die miteinander einen Winkel zwischen 0° und 180°, Grenzen ausgeschlossen, bilden, wobei diese beiden ebenen Flächen (Sf1, Sf2) in die rotationssymmetrische Hülle, die durch die äußere Oberfläche des ersten Teils (221) des zweiten Rohrs (22) definiert ist, aufgenommen sind.

5. Vorrichtung nach Anspruch 4, **dadurch gekennzeichnet, dass** die beiden im Wesentlichen ebenen Flächen (Sf1, Sf2) miteinander einen Winkel im Wesentlichen gleich 90° bilden.

6. Vorrichtung nach einem der Ansprüche 4 und 5, **dadurch gekennzeichnet, dass** sie zwei isolierte Leiter (31, 32) umfasst, die durch den Clip (41) an jeweils einer der beiden im Wesentlichen ebenen Flächen (Sf1, Sf2) in Kontakt gehalten werden.

7. Vorrichtung nach einem der vorhergehenden Ansprüche, **dadurch gekennzeichnet, dass** sie ein drittes zylindrisches Rohr (50) umfasst, wobei dieses dritte Rohr umfasst:
• einen ersten rotationssymmetrischen zylindrischen Teil (501) mit einem Zentrumswinkel mit einem Wert im Wesentlichen gleich 180°, und einem Außenradius mit Wert R3, der im Wesentlichen gleich dem Wert des Radius der rotationssymmetrischen zylindrischen inneren Oberfläche des ersten Teils (221) des zweiten Rohrs (22) ist,
• einen im Wesentlichen ebenen zweiten Teil (502), wobei die beiden Enden dieses zweiten Teils des dritten Rohrs in die beiden entsprechenden Enden des ersten Teils (501) des dritten Rohrs übergehen,
• wobei das dritte Rohr (50) im Inneren des zweiten Rohrs (22) derart angeordnet ist, dass sein erster Teil (501) mit der inneren Oberfläche des ersten Teils (221) des zweiten Rohrs (22) im Wesentlichen kongruent ist und dieser entspricht.

8. Vorrichtung nach Anspruch 7, wenn abhängig von Anspruch 5, **dadurch gekennzeichnet, dass** sie ein zylindrisches, gegebenenfalls rotationssymmetrisches viertes Rohr (60) umfasst, das in dem Raum (61) angeordnet ist, der zwischen dem im Wesentlichen ebenen zweiten Teil (502) des dritten Rohrs (50) und den beiden ebenen Flächen (Sf1, Sf2) des zweiten Rohrs (22) definiert ist.

9. Vorrichtung nach einem der Ansprüche 7 und 8, **dadurch gekennzeichnet, dass** sie ein fünftes Rohr (70) umfasst, das in dem dritten Rohr (50) montiert ist.

10. Vorrichtung nach den Ansprüchen 1 und 7, **dadurch gekennzeichnet, dass** das erste Rohr (21), der erste Teil (221) des zweiten Rohrs (22) und der erste Teil (501) des dritten Rohrs (50), die alle drei rotationssymmetrisch zylindrisch sind, im Wesentlichen koaxial (Ax) sind.

## Claims

1. A device for performing resection of an organ in a cavity of a living body, in particular of a human being, known by the term "resectoscope", and comprising at least:
• a guide (10) defined between a proximal end (11) and a distal end (12), said guide defining a duct (13) that is open at both its proximal end and its distal end, said guide being constituted at least by a first cylindrical tube (21) and by a second cylindrical tube (22), the second tube (22) being mounted in the first tube (21);
• at least one electrical conductor surrounded by a sheath (33, 34) made of electrically insulating material, said electrical conductor as insulated in this way (31, 32) being mounted in said duct (13); and
• inlet/outlet means (14) mounted in cooperation with the proximal end (11) of said guide (10);
the device being **characterized by** the facts that:
• said first tube (21) is a cylinder of revolution having an inside radius of value R1;
• said second tube (22) comprises:
* a first portion (221) being a portion of a cylinder of revolution having a center angle of value Ac and an outside radius of value R2, where R2 is less than R1; and
* a second portion (222) that is a portion of a cylinder that is not a cylinder of revolution and having an angle at the center of value "360° - Ac", the two edges (222-1, 222-2) of the second portion (222) of the second tube (22) being in continuity respectively with the two edges of the first portion (221) of the second tube;
• said insulated conductor (31, 32) being mounted in contact on said second portion (222) of said second tube (22) between the outside wall of the second tube and the inside wall of the first tube (21); and
• that it further includes means (40) for holding said insulated conductor (31, 32) in contact on said second portion (222) of the second tube (22).

2. A device according to claim 1, **characterized by** the fact that the means (40) for holding said insulated conductor (31, 32) in contact on the second portion (222) of the second tube (22) are constituted by a clip (41) having two lateral longitudinal edges, each in the form of a re-entrant resilient hook (42, 43), the two re-entrant resilient hooks being clipped by going over the two connection edges (A1, A2) formed respectively between the first portion (221) of the second tube (22) and its second portion (222), and wrapping at least in part around these two edges, said insulated conductor (31, 32) being positioned between the clip (41) and said second portion (222) of the second tube (22).

3. A device according to claim 1 or claim 2, **characterized by** the fact that said second portion (222) of the second tube (22) presents at least one substantially plane surface (Sf1, Sf2) facing the inside wall of the first tube (21).

4. A device according to claim 3, **characterized by** the fact that said second portion (222) of the second tube (22) presents two substantially plane surfaces (Sf1, Sf2) facing the inside wall of the first tube, these two plane surfaces (Sf1, Sf2) forming between them an angle lying between 0° and 180°, bounds not included, and being inscribed in the surface of revolution defined by the outside surface of the first portion (221) of the second tube (22).

5. A device according to claim 4, **characterized by** the fact that the two substantially plane surfaces (Sf1, Sf2) make between them an angle that is substantially equal to 90°.

6. A device according to claim 4 or claim 5, **characterized by** the fact that it includes two insulated conductors (31, 32) held by said clip (41) in contact respectively on each of the two substantially plane surfaces (Sf1, Sf2).

7. A device according to any preceding claim, **characterized by** the fact that it includes a third cylindrical tube (50), said third tube comprising:
• a first portion (501) forming a portion of a cylinder of revolution with a center angle having a value substantially equal to 180° and an outside radius of value R3 substantially equal to the value of the radius of the cylindrical inside surface of revolution of the first portion (221) of the second tube (22);
• a substantially plane second portion (502), the two edges of the second portion of the third tube being in continuity respectively with the two edges of said first portion (501) of said third tube;
• said third tube (50) being arranged inside the second tube (22) in such a manner that its first portion (501) is substantially congruent and in correspondence with the inside surface of the first portion (221) of the second tube (22).

8. A device according to claim 7, when dependent claim 5, **characterized by** the fact that it includes a fourth cylindrical tube (60) forming a cylinder optionally of revolution, situated in the space (61) defined between the substantially plane second portion (502) of the third tube (50) and the two plane surfaces (Sf1, Sf2) of the second tube (22).

9. A device according to claim 7 or claim 8, **characterized by** the fact that it includes a fifth tube (70) mounted in said third tube (50).

10. A device according to claims 1 and 7, **characterized by** the fact that said first tube (21), the first portion (221) of the second tube (22), and the first portion (501) of the third tube (50), all three of which are in the shape of portions of cylinders of revolution, share a substantially common axis (Ax).
